(19) Europäisches Patentamt
European Patent Office
Office européen des brevets

(11) Publication number : **0 639 376 A1**

(12) **EUROPEAN PATENT APPLICATION**

(21) Application number : **94305890.9**

(22) Date of filing : **09.08.94**

(51) Int. Cl.$^6$ : **A61K 31/445**, A61K 47/26, A61K 47/44

(30) Priority : **18.08.93 JP 204149/93**

(43) Date of publication of application :
**22.02.95 Bulletin 95/08**

(84) Designated Contracting States :
**AT BE CH DE DK ES FR GB GR IE IT LI LU MC NL PT SE**

(71) Applicant : **TERUMO KABUSHIKI KAISHA**
**No. 44-1, Hatagaya 2-chome,**
**Shibuya-ku**
**Tokyo 151 (JP)**

(72) Inventor : **Kimoto, Tomoaki**
**30-106 Hanesawa,**
**695-3 Endou**
**Fujisawa, Kanagawa (JP)**
Inventor : **Konno, Ryouya**
**105 Haitsu Hayama,**
**1335-15 Minami-Oya**
**Machida-shi, Tokyo (JP)**

(74) Representative : **Harrison, David Christopher et al**
**MEWBURN ELLIS**
**York House**
**23 Kingsway**
**London WC2B 6HP (GB)**

(54) **Liquid composition containing amide derivative.**

(57) The present invention is an aqueous liquid composition comprising an anti-allergic amide derivative of which the solubility has been increased by the presence of a nonionic surfactant and a pH adjusting agent so that the composition has a pH of from 4 to 5. Solubility of the amide is further increased if the amount of surfactant is at least 10 times that of the amide and its stability is improved if the amount of surfactant is 0.1 W/V % or more. The composition finds use as eye or nose drops in the treatment of allergic conditions.

EP 0 639 376 A1

The present invention relates to an aqueous liquid composition having a particular amide derivative stably dissolved therein.

Amide derivatives described in US-A-4,795,752, are useful as active ingredients in anti-allergic agents. These are stated to be applicable to collunaria (nasal douches or sprays) and to eye drops for curing pollinosis (hay fever) etc.

Those derivatives are of the formula (I):

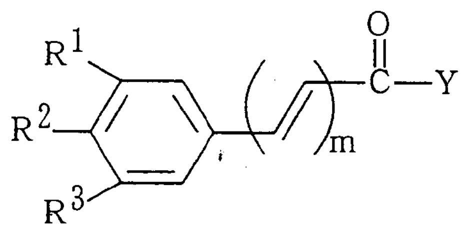

(I)

wherein $R^1$ represents a lower alkoxyl group or a lower alkoxycarbonyloxy group, $R^2$ represents a lower alkoxycarbonyloxy group, $R^3$ represents a hydrogen atom, m represents an integer of 1 or 2, in which *lower* is defined as having 1 to 5 carbon atoms, and Y represents a group represented by the formula (II):

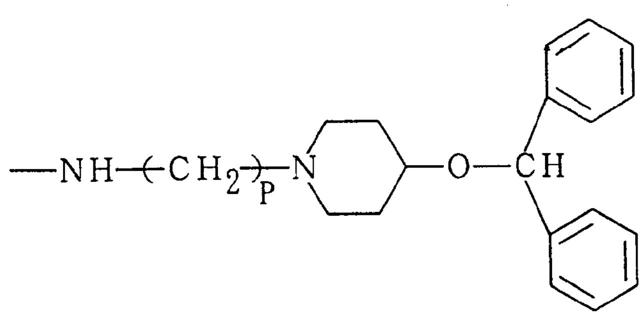

(II)

wherein p represents an integer of from 2 to 4.

Where the amide derivatives are to be administered to patients suffering from allergic rhinitis or allergic ophthalmia as liquid collunaria or eye drops, it is said that the concentration of the amide derivative in the liquid agents must be from 0.005 to 0.5 W/V %.

However, the amide derivatives are virtually insoluble in water, and it was extremely difficult to prepare aqueous solutions having the necessary concentration of the derivative. In addition, since the amide derivatives are easily hydrolyzed in their aqueous solutions, there is a problem of how to stabilize them in aqueous solution.

In consideration of the above-mentioned problems, the object of the present invention is to provide liquid compositions containing the amide derivatives where the amide derivatives are stable therein without losing their pharmaceutical properties.

The present inventors investigated the solubilities of the amide derivatives in various aqueous solutions by various means and, as a result, have found that their solubilities in aqueous solutions are noticeably increased when a nonionic surfactant having HLB of from 10 to 16, preferably from 13 to 16 is added thereto as a solubilizer along with an organic acid and/or an organic acid buffer while the pH value of the solutions is adjusted to within the range of from 4.0 to 5.0. In order to sufficiently dissolve the amide derivatives in the weight of the nonionic surfactant to be added thereto should be 10 times or more than the weight of the amide derivative therein. In order to obtain stable liquid compositions containing the amide derivatives where the hydrolysis of the amide derivatives is inhibited by the protective effect of micelles formed therein, the amount of the nonionic surfactant to be in the composition should be 0.1 W/V % or more. On the other hand, however, it is desirably 5 W/V % or less, considering that the liquid compositions may be used as eye drops or collunaria and they are desired not to stimulate mucous membranes.

The nonionic surfactant usable in the present invention includes, for example, polyoxyethylene-hardened castor oil, polyoxyethylene-sorbitan monooleate, polyoxyethylene-nonylphenyl ether, polyoxyethylene-cetyl ether, polyoxyethylene-glycol monostearate, etc. Of these, most preferred are polyoxyethylene-hardened castor oil (HCO-60) and polyoxyethylene-sorbitan monooleate (Tween 80), as they are of low toxicity.

The aqueous compositions of the present invention are used at a pH of from 4.0 to 5.0, because of the solubility and the stability of the amide derivatives therein. To adjust the pH value of the compositions, various organic acids and/or organic acid buffers may be used. Examples are citric acid, tartaric acid, lactic acid, acetic acid and buffers containing them.

The lactic acid buffers are most preferred among them because of the properties which are odorless and improve the solubility of the amide derivatives.

It is preferred that the concentration of the above-mentioned organic acids and/or organic acid buffers in the aqueous compositions falls within the range of from 0.05 mol/liter to 1.0 mol/liter. Where the aqueous compositions are used as eye drops or collunaria, however, they are desired to be isosmotic with the body as much as possible in order that the compositions do not stimulate the mucous membrane. In this case, therefore, the concentration is more preferably from 0.1 to 0.3 mol/liter.

The aqueous preparations of the present invention may be used as various anti-allergic collunaria, eye drops, endermic agents, inhalants, internal medicines and injections.

Liquid carriers to be used for dissolving the above-mentioned amide derivatives, non-ionic surfactants and organic acids so as to prepare the liquid compositions of the present inventions are optional, and distilled water, pure water, physiological saline solution and others that are ordinarily used in this technical field may be employed.

The aqueous compositions of the present invention may optionally contain, in addition to the above-mentioned ingredients, various additives which are generally used to prepare ordinary aqueous compositions, such as colorants, flavorings, vehicles, disintegrators, lubricants, tackifiers, preservatives, aromatics, propellants, etc.

The aqueous compositions of the present invention may be prepared by various methods generally employed for preparing ordinary liquid compositions.

For instance, they may be prepared by dissolving under heat the above-mentioned amide derivative in a small amount of distilled water along with a nonionic surfactant followed by adding a pH-adjusting buffer thereto so as to completely dissolve the derivative in the aqueous solution.

The present invention will be explained in more detail hereunder, with reference to the following examples.

In the following examples, 1-[{5'-(3"-methoxy-4"-ethoxycarbonyloxyphenyl)-2',4'-pentadienoyl}aminoethyl]-4-diphenylmethoxypiperidine of the following formula (III) was used as the amide derivative.

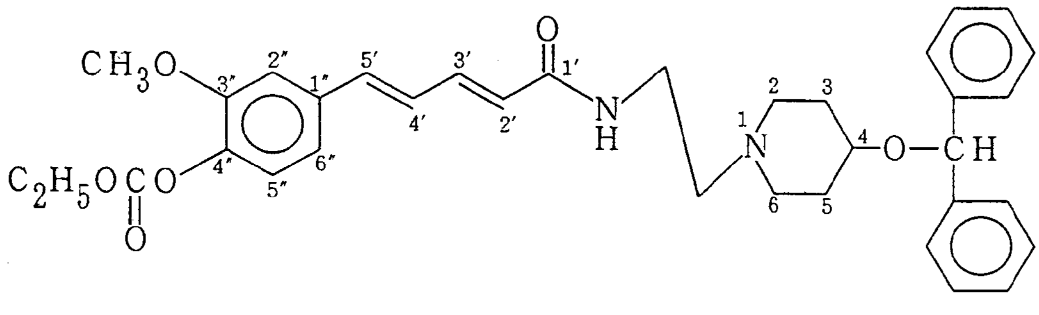

(III)

The above-mentioned compound is a white or pale yellow, crystalline powder having a melting point of 100°C. This is easily soluble in dichloroethane and acetone, soluble in acetonitrile to some degree, hardly soluble in ethanol and almost insoluble in water.

Test Example 1: Test for Solubility

To test the solubility of the above-mentioned compound, various experiments were carried out under different conditions in the presence or absence of a nonionic surfactant.

Formulation No. 1 was prepared by heating and dissolving 0.05 g of Compound (III) and 0.5 g of polyoxyethylene-hardened castor oil (HCO-60) in a beaker at about 60°C followed by gradually adding thereto lactic acid/sodium lactate buffer (pH 5.0) as the pH buffer while stirring to dissolve them.

The other formulations in Table 1 below were prepared in the same manner as above, whereupon the dissolution of the compound was observed with the naked eye. The results are shown in Table 1.

Table 1

| Formulation No. | 1-1 | 1-2 | 1-3 | 1-4 | 1-5 | 1-6 |
|---|---|---|---|---|---|---|
| Compound (III) (g) | 0.05 | 0.05 | 0.05 | 0.05 | 0.05 | 0.05 |
| HCO-60 (g) (*1) | 0.5 | 1.0 | 1.5 | | | |
| Tween 80 (g) (*2) | | | | 0.5 | 1.0 | 1.5 |
| Propylene Glycol | | | | | | |
| pH Buffer (ml) (*3) | ad lib. (*5) | ad lib. | ad lib. | ad lib. | ad lib. | ad lib. |
| Pure Water (ml) | | | | | | |
| Result | Dissolved clear. | Dissolved clear. | Dissolved clear. | Dissolved clear. | Dissolved clear. | Dissolved clear. |

Table 1 (continued)

| Formulation No. | 1-7 | 1-8 | 1-9 | 1-10 |
|---|---|---|---|---|
| Compound (III) (g) | 0.05 | 0.05 | 0.05 | 0.05 |
| HCO-60 (g) (*1) | | 1.0 | | 1.0 |
| Tween 80 (g) (*2) | | | | |
| Propylene Glycol | 40 | | | |
| pH Buffer (ml) (*3) | ad lib. | | ad lib. | |
| pH Buffer (ml) (*4) | | | | ad lib. |
| Pure Water (ml) | | ad lib. | | |
| Result | Dissolved clear. | Not dissolved. | Not dissolved. | Not dissolved. |

(*1) Polyoxyethylene-hardened castor oil

(*2) Polyoxyethylene-sorbitan monooleate

(*3) 1.0 mol/liter lactic acid-sodium lactate buffer (pH 5.0)

(*4) 0.05 mol/liter potassium dihydrogen phosphate-disodium hydrogen phosphate buffer (pH 5.0)

(*5) To make 100 ml in total.

Comparing Formulations (1-1) to (1-6) with Formulations (1-8) to (1-10) in Table 1 above, it is noted that the combination of the nonionic surfactant and the organic acid buffer results in excellent dissolution of Compound (III). Putting only the solubility of Compound (III) in question, it is noted that the compound may be dissolved clear in the presence of some solubilizer (propylene glycol) other than nonionic surfactants (see Formulation (1-7)).

Test Example 2: Test for Stability

The difference in the stability of Compound (III) was tested, depending on the solubilizer added thereto. The stability was represented by the percentage (%) of the amount of Compound (III) remained after storage. Precisely, each sample formulation shown in Table 2 below was put in a glass tube with a screw cap and stored for 10 days and 20 days both at 60°C, whereupon the percentage of the amount of the compound remained after the storage was measured by liquid chromatography. From the value thus measured, the stability of the compound was evaluated. The results are shown in Table 2.

Table 2

| Formulation No. | 2-1 | 2-2 | 2-3 | 2-4 | 2-5 | 2-6 | 2-7 |
|---|---|---|---|---|---|---|---|
| Compound (III) (g) | 0.05 | 0.05 | 0.05 | 0.05 | 0.05 | 0.05 | 0.05 |
| HCO-60 (g) | 0.5 | 1.0 | 1.0 | 1.5 | | | |
| Tween 80 (g) | | | | | 0.5 | 1.0 | 1.0 |
| Propylene Glycol (g) | | | | | | | |
| Buffer 1 (ml) (*1) | ad lib. (*3) | ad lib. | | ad lib. | | | ad lib. |
| Buffer 2 (ml) (*2) | | | ad lib. | | ad lib. | ad lib. | |
| After stored for 10 days at 60°C | 92.3 | 93.8 | 95.1 | 96.2 | 89.8 | 91.8 | 89.9 |
| After stored for 20 days at 60°C | 90.5 | 92.0 | 92.7 | 92.5 | 87.1 | 89.7 | 90.5 |

Table 2 (continued)

| Formulation No. | 2-8 | 2-9 | 2-10 |
|---|---|---|---|
| Compound (III) (g) | 0.05 | 0.05 | 0.05 |
| HCO-60 (g) | | | |
| Tween 80 (g) | 1.5 | | |
| Propylene Glycol (g) | | 40 | 40 |
| Buffer 1 (ml) (*1) | | | ad lib. |
| Buffer 2 (ml) (*2) | ad lib. | ad lib. | |
| After stored for 10 days at 60°C | 92.0 | 73.7 | 81.9 |
| After stored for 20 days at 60°C | 91.4 | 58.6 | 65.3 |

(*1) 1.0 mol/liter lactic acid/sodium lactate buffer (pH 5.0)

(*2) 1.0 mol/liter lactic acid/sodium lactate buffer (pH 4.0)

(*3) To make 100 ml in total.

From Table 2 above, it is noted that the aqueous compositions of the present invention (Formulations (2-1) to (2-8)) were stabilized due to the protecting activity of the micelles formed therein. However, the different solubilizer (propylene glycol) than nonionic surfactants was not effective in stabilizing Compound (III) (Formulations (2-9) and (2-10)), though having no problem about the solubility of the compound (Test Example 1).

Next, to examine the influence of the concentration of the solubilizer on the stability of Compound (III), experiments were carried out where the concentration of the nonionic surfactant to be added was stepwise increased while the concentration of Compound (III) was kept constant. Precisely, each sample formulation shown in Table 3 below was stored for 10 days and 20 days both at 60°C, whereupon the percentage (%) of the amount of Compound (III) remained after the storage was measured by liquid chromatography. On the basis of the thus-measured percentage, the stability of the compound was evaluated. The results are shown in Table 3.

Table 3

| Formulation No. | 2-11 | 2-12 | 2-13 | 2-14 | 2-15 |
|---|---|---|---|---|---|
| Compound (III) (g) | 0.005 | 0.005 | 0.005 | 0.005 | 0.005 |
| HCO-60 (g) | 0.05 | 0.10 | 0.25 | 0.50 | 1.00 |
| pH Buffer (ml)(*3) | ad lib. (*) | ad lib. (*) | ad lib. (*) | ad lib. (*) | ad lib. (*) |
| Stored for 10 days at 60°C | 75.2 | 84.3 | 93.2 | 91.9 | 94.7 |
| Stored for 20 days at 60°C | 56.4 | 70.0 | 81.7 | 84.1 | 86.4 |

(Note) The data indicate the percentage (%) of the amount of Compound (III) remained after the storage.

(*3) This is 1.0 mol/liter lactic acid-sodium lactate buffer (pH 5.0).

(*) To make 100 ml in total.

From Table 3 above, it is noted that the stability of Compound (III) increased with increase of the concentration of HCO-60 added and that the compound was almost satisfactorily stabilized due to the protecting effect of the micelled surfactant when the concentration of the surfactant HCO-60 added was 0.10 W/V % or more.

Table 4 below shows the mean particle size of the formed micelles, measured by light-scattering photometry. It was confirmed that, when the concentration of Compound (III) was 0.005 W/V % and when the concentration of HCO-60 was 0.10 W/V % or more, micelles having a mean particle size of from 16 to 20 nm were formed.

Table 4

| Concentration of Compound (III) (W/V %) | Concentration of HCO-60 (W/V %) | Mean Particle Size (nm) |
|---|---|---|
| 0.005 | 0.05 | - |
| 0.005 | 0.10 | 16.2 +/- 3.7 |
| 0.005 | 0.25 | 19.6 +/- 4.3 |
| 0.005 | 0.50 | 18.3 +/- 4.5 |
| 0.005 | 1.00 | 18.8 +/- 4.2 |

- : No micelle detected.

The following Compounds (IV), (V), (VI) and (VII) were tested in the same manner as above, with respect to their solubility and stability. The results are shown in Table 5 below.

(IV)

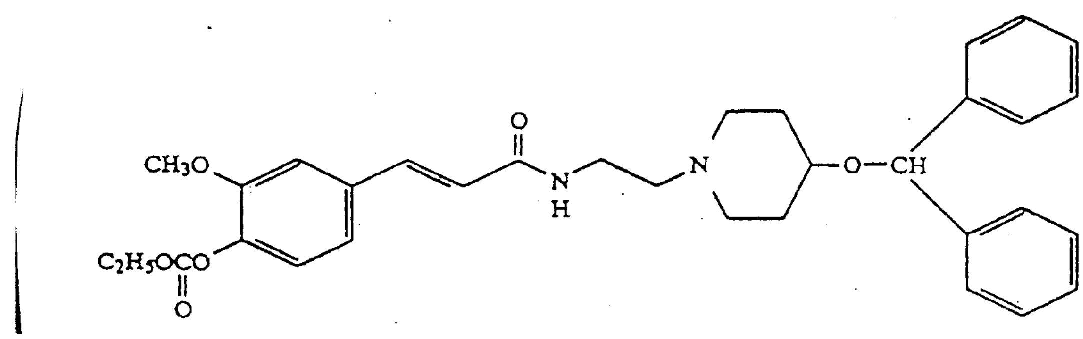

(V)

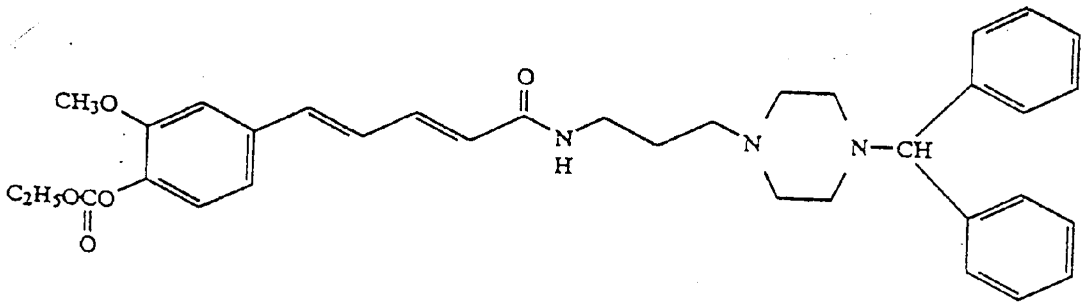

(VI)

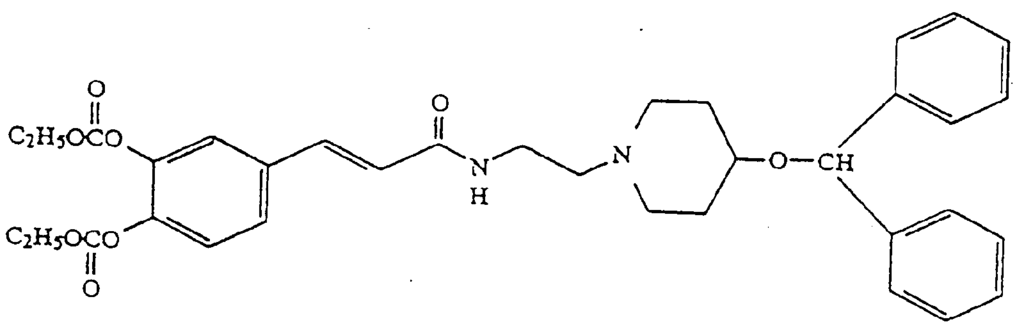

(VII)

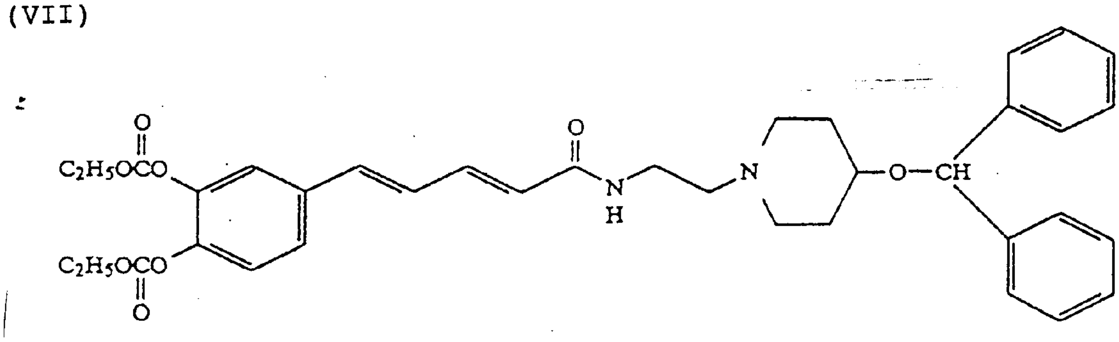

Table 5

| Compound | (IV) | (V) | (VI) | (VII) |
|---|---|---|---|---|
| Compound (g) | 0.05 | 0.05 | 0.05 | 0.05 |
| HCO-60 (g) | 1.0 | 1.0 | 1.0 | 1.0 |
| pH Buffer (ml) (*1) | ad lib. (*2) | ad lib. | ad lib. | ad lib. |
| Solubility | Dissolved clear. | Dissolved clear. | Dissolved clear. | Dissolved clear. |
| Stability (as percentage of compound remained after stored at 60°C for 20 days) | 91.2 | 92.1 | 90.9 | 92.6 |

(*1) 1.0 mol/liter lactic acid-sodium lactate buffer (pH 5.0)

(*2) To make 100 ml in total.

Test Example 3: Test for Pharmaceutical Activity against Experimental Allergic Rhinitis:

Using male Hartley guinea pigs as test animals for dye-bleeding models, Compound (III) was tested to determine its pharmaceutical activity. Precisely, the test animals were subjected to thoracotomy while anesthetized, a polyethylene tube was inserted into the trachea of each animal, a dye (4 % Brilliant Blue, 1 mg/kg) was intravenously injected to each animal, one of aqueous preparations (Formulations (3-1) to (3-3)) of the present invention and comparative aqueous preparations (Formulations (3-4) and (3-5)) was pre-administered into the nasal cavity of each animal through the tube, and a histamine solution was administered into the nasal cavity thereof in the same manner as above, whereupon the dye running out from the blood vessels in the nasal cavity due to the allergic reaction in the test animal was quantitatively determined by absorptiometry. The results are shown in Table 6.

Table 6

| Formulation No. | 3-1 | 3-2 | 3-3 | 3-4 | 3-5 (*5) |
|---|---|---|---|---|---|
| Compound (III) (g) | 0.005 | 0.05 | 0.5 | - | 0.05 |
| HCO-60 (g) | 0.05 | 0.5 | 5.0 | - | - |
| pH Buffer (ml) (*3) | ad lib. (*) | ad lib. (*) | ad lib. (*) | ad lib. (*) | - |
| Pure Water (ml) | - | - | - | - | ad lib. (*) |
| Amount of Dye (μg) running out from test animal for 30 minutes | 11.1 +/-3.8 | 5.9 +/-2.6 | 14.0 +/-2.6 | 20.4 +/-9.2 | 21.5 +/-7.3 |

(*3) 1.0 mol/liter lactic acid-sodium lactate buffer (pH 5.0)

(*5) Suspension

(*) To make 100 ml in total.

The amount of the dye running out from the nasal cavity of the test animal, to which one of Formulations (3-1) to (3-3) had been administered, was reduced. In these test animals, the significant pharmaceutical activity

of Compound (III) was admitted. However, no pharmaceutical activity was admitted, when the suspension of Compound (III) (Formulation (3-5)) was administered. These results thus verified the solubilizing effect attainable by the combination of HCO-60 and the pH buffer.

Formulation Examples:

Formulation examples of aqueous preparations containing the amide derivative of the present invention are illustrated below.

Formulation of Collunarium 1:

| | |
|---|---|
| Compound (III) | 0.5 g |
| HCO-60 | 5.0 g |
| Benzyl Alcohol | 9.0 g |
| 0.1 mol/liter lactic acid-sodium lactate buffer (pH 5.0) | ad lib. |
| | 1000 ml in total |

How to prepare collunarium 1:

Lactic acid was added to pure water and dissolved, and the resulting solution was adjusted to have pH of 5.0 by adding sodium hydroxide thereto. Apart from this, Compound (III) and HCO-60 were weighed and dissolved under heat. To this were added benzyl alcohol and the previously-prepared lactic acid buffer and dissolved while stirring under heat. After cooled, the solution was filtered through a 0.45 μm-membrane filter and filled in a pre-determined container. Thus prepared was a liquid composition for collunarium.

Formulation of Collunarium 2:

| | |
|---|---|
| Compound (IV) | 0.05 g |
| Tween 80 | 2.0 g |
| Methyl P-hydroxybenzoate | 0.3 g |
| Propyl P-hydroxybenzoate | 0.1 g |
| 0.2 mol/liter acetic acid-sodium acetate buffer (pH 5.0) | ad lib. |
| | 1000 ml in total |

How to prepare collunarium 2:

Acetic acid was added to pure water and dissolved, and the resulting solution was adjusted to have pH of 5.0 by adding sodium hydroxide thereto. Apart from this, Compound (IV) and Tween 80 were weighed and dissolved under heat. To this were added methyl p-hydroxybenzoate, propyl p-hydroxybenzoate and the previously-prepared acetic acid buffer and dissolved while stirring under heat. After cooled, the solution was filtered through a 0.45 μm-membrane filter and filled in a pre-determined container. Thus prepared was a liquid composition for collunarium.

Formulation of Collunarium 3:

| | |
|---|---|
| Compound (V) | 0.05 g |
| HCO-60 | 1.0 g |
| Benzalkonium Chloride | 0.1 g |
| 0.3 mol/liter lactic acid-sodium lactate buffer (pH 4.0) | ad lib. |
| | 1000 ml in total |

How to prepare collunarium 2:

Lactic acid was added to pure water and dissolved, and the resulting solution was adjusted to have pH of 4.0 by adding sodium hydroxide thereto. Apart from this, Compound (V) and HCO-60 were weighed and dissolved under heat. To this were added phenylethyl alcohol and the previously-prepared lactic acid buffer and dissolved while stirring under heat. After cooled, the solution was filtered through a 0.45 μm-membrane filter and filled in a pre-determined container. Thus prepared was a liquid composition for collunarium.

Formulation of Eye Drops:

| | |
|---|---|
| Compound (III) | 0.5 g |
| HCO-60 | 5.0 g |
| Sodium Azulenesulfonate | 0.2 g |
| Benzalkonium Chloride | 0.1 g |
| 0.15 mol/liter tartaric acid-sodium tartrate buffer (pH 4.0) | ad lib. |
| | 1000 ml in total |

How to prepare dye drops:

Tartaric acid was added to pure water and dissolved, and the resulting solution was adjusted to have pH of 5.0 by adding sodium hydroxide thereto. Apart from this, Compound (III) and HCO-60 were weighed and dissolved under heat. To this were added sodium azulenesulfonate, benzalkonium chloride and the previously-prepared tartaric acid buffer and dissolved while stirring under heat. After cooled, the solution was filtered through a 0.45 μm-membrane filter and filled in a pre-determined container. Thus prepared was a liquid composition for eye drops.

Formulation of Inhalant:

| | |
|---|---|
| Compound (III) | 0.05 g |
| HCO-60 | 1.0 g |
| Chlorobutanol | 5.0 g |
| 0.2 mol/liter lactic acid-sodium lactate buffer (pH 5.0) | ad lib. |
| | 1000 ml in total |

How to prepare inhalant:

Lactic acid was added to pure water and dissolved, and the resulting solution was adjusted to have pH of 5.0 by adding sodium hydroxide thereto. Apart from this, Compound (III) and HCO-60 were weighed and dissolved under heat. To this were added chlorobutanol and the previously-prepared lactic acid buffer and dissolved while stirring under heat. After cooled, the solution was filtered through a 0.45 μm-membrane filter and filled in a pre-determined container. Thus prepared was a liquid composition for inhalant.

All the preparations prepared in the above-mentioned Formulation Examples showed excellent solubility, stability and anti-allergic activity, like the aqueous preparations of the present invention tested in the foregoing Test Examples.

## Claims

1. An aqueous liquid composition comprising an amide derivative represented by the following formula (I),

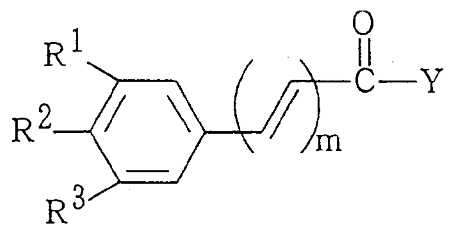

(I)

wherein $R^1$ represents a lower alkoxyl group or a lower alkoxycarbonyloxy group, $R^2$ represents a lower alkoxycarbonyloxy group, $R^3$ represents a hydrogen atom, m represents an integer of 1 or 2, "lower" represents $C_1$ to $C_5$ and Y represents a group represented by the formula (II):

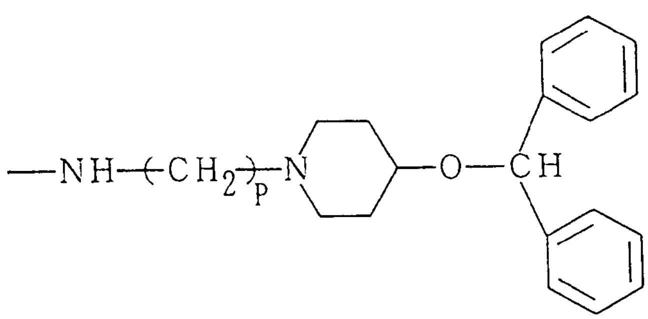

(II)

wherein p represents an integer of from 2 to 4,

characterized in that the composition includes a nonionic surfactant having an HLB of from 10 to 16 and has a pH of 4 to 5.

2. The aqueous liquid composition as claimed in claim 1, in which the amount of the nonionic surfactant is 10 times or more than that of the amide derivative.

3. The aqueous liquid composition as claimed in claim 1 or claim 2, in which the concentration of the nonionic surfactant is 5 W/V % or less.

4. The aqueous liquid composition as claimed in claim 1, claim 2 or claim 3, in which the nonionic surfactant is a polyoxyethylene-hardened castor oil.

5. The aqueous liquid composition as claimed in claim 1, claim 2 or claim 3, in which the nonionic surfactant is a polyoxyethylene-sorbitan monooleate.

6. The aqueous liquid composition as claimed in any one of the preceding claims, in which the pH is adjusted

to 4 to 5 by an organic acid or organic acid buffer.

7. The aqueous liquid composition as claimed in claim 6, in which the pH adjusting agent is a lactic acid buffer.

8. The use of an aqueous liquid composition as claimed in any one of the preceding claims, in the manufacture of a medicament for the treatment of an allergic condition.

9. The use as claimed in claim 8, wherein the medicament is a nose or eye drop or spray.

European Patent Office

# EUROPEAN SEARCH REPORT

Application Number
EP 94 30 5890

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A | EP-A-0 537 070 (TERUMO KABUSHIKI KAISHA)<br>* claims *<br>* page 6, line 21 - line 34 *<br>--- | 1-9 | A61K31/445<br>A61K47/26<br>A61K47/44 |
| D,A | EP-A-0 226 516 (TERUMO CORPORATION)<br>* claims *<br>* page 3, line 34 - line 42 *<br>--- | 1-9 | |
| A | CHEMICAL ABSTRACTS, vol. 111, no. 12, 18 September 1989, Columbus, Ohio, US;<br>abstract no. 102701g,<br>* abstract *<br>& WO-A-88 04169 (TERUMO CORPORATION) 16 June 1988<br>----- | 1-9 | |
| | | | TECHNICAL FIELDS SEARCHED (Int.Cl.6) |
| | | | A61K |

The present search report has been drawn up for all claims

| Place of search | Date of completion of the search | Examiner |
|---|---|---|
| THE HAGUE | 16 November 1994 | Scarponi, U |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)